# EUROPEAN PATENT APPLICATION

(11) **EP 2 669 829 A1**
(43) Date of publication of application: **04.12.2013**
(21) Application number: 12170304.5
(22) Date of filing: 31.05.2012
(51) Int. Cl.: G06F 19/00

(54) **Method and medical system for controlled centralised distribution of prescriptions**

(71) Applicant: Belintra nv, 9890 Semmerzake (BE)
(72) Inventor: Schepens, Geert, 9890 Semmerzake (BE)
(74) Representative: Gyi, Jeffrey Ivan

(57) **Abstract**

The present invention provides a method and system for controlled distribution of prescriptions using a system **100** of patient-assigned containers **10** each with a wirelessly-controlled programmable display and wireless communication units **30** to individually program and change the display. The patient-assigned containers **10** stay in communication with the wireless communication units **30,** to assist the administering nurse in the choosing the correct container at the patient bedside, and to warn of prescription changes.

## Description

### FIELD OF THE INVENTION

The present invention is in the field of controlled distribution of medical prescriptions within a health care centre, more in particular, to a patient-specific medical container provided with an electronic display, adapted to receive wireless instructions to display information selectively, so maintaining the privacy and safety of the patient.

### BACKGROUND TO THE INVENTION

Prescriptions, which comprise medications, and other medicals supplies such as syringes are currently distributed from a central location in a healthcare centre, such as from a central pharmacy to specific patients. Distribution is facilitated by the use of medication containers. Into the medication container is placed medication or other medical supplies by the pharmacist or an authorised assistant that are to be provided to a particular patient. Typically, the medical container holds sufficient medication for one day or other time frame. The medication containers disposed with suitable supplies is transported to the destination, such as to the ward, typically on a medication trolley. At the destination, the medication container assigned to the patient is withdrawn from the trolley and the medication administered. The medication container may be returned to the trolley, until the next scheduled administration.

The medication and other medicals supplies should be delivered to the correct patient. Labels are generally printed or handwritten with the name of the patient and the patient's location, such as the unit, floor and/or room number of the patient. Also electronic displays are used to display the information, so allowing the convenient recycling of a medical container. Medical containers may be loaded using a robotic dispensing system, capable of loading hundreds of medical containers in a day.

However, one problem in the art lies in patient confidentiality. The information indicated on the container shows the patient's name, and from the contents of the container, anyone can ascertain the condition suffered by the patient whether authorised or not. The system of the art therefore compromises patient confidentiality.

A further problem in the art lies in patient safety. While information on the container shows the patient's name, a nurse may inadvertently select the wrong container, particularly when two or more patients share the same or a similar name.

Another problem in the art lies in managing changes to a prescription by a doctor which arise after the medical container has left the pharmacy. Medical containers are often loaded several hours prior to administration, during which time a doctor may adjust a dose or prescription to respond to changes in the patient condition. The system of the art does is not flexible to account for such changes, that are known as 'deltas'.

The present invention aims to overcome one or more of these problems in the art.

### SUMMARY OF THE INVENTION

One embodiment of the invention is a method (200) for computer assisted distribution of a medical prescription for a patient within a healthcare centre comprising the steps:
a) obtaining patient-specific data (202);
b) sending (204) patient-specific data (202) to at least one wireless communication unit (30) configured to transmit one or more wireless signals specific to a patient-assigned container (10), which patient-assigned container (10) comprises:
   i. a housing (12) that defines a cavity (14) for receiving one or more prescriptions items, and
   ii. a wireless-activated readable display (20) attached to the housing (12), configured to:
      - receive and erasably store the patient-specific data, and
      - selectively display information,
         responsive to one or more wireless signals (22) specific to the patient-specific container (10);
c) sending (208) instruction (206) to at least one wireless communication unit (30) to transmit a wireless signal to effect displaying of non-patient-specific data (22) by the readable display (20);
d) receiving (210) patient identification data (212);
e) sending (220) instruction (222) to at least one wireless communication unit (30) to effect displaying of patient-specific data (24) by the readable display (20) of the container (10) that has been assigned to the patient identified by the identification data (212); thereby allowing a healthcare professional to confirm and identify the patient-assigned-container allotted to the patient prior to administration of the prescription items.

The patient-assigned container (10) may be identified using a container unique identification tag, CUID tag (19), attached to the container (10), and which is assigned to the patient.

The CUID tag may be a 1 D or 2D barcode, or a radiofrequency identification tag.

The patient specific data may be displayed in step e) comprises the patient name, and one or more of hospital building, hospital ward, room number, bed number, and date of birth.

The method may further comprise a step after step c) of receiving an indication that the prescription of the patient has changed, and wherein the patient-specific data (24) displayed in step e) is a stored alert message.

The patient identification data (212) may be obtained in step d) using:
- a scanner (214) configured to read a patient identification tag, PID tag, (216) disposed on or in the patient (218),
- face recognition,
- finger print recognition, or
- manual input by a healthcare professional.

The method may further comprise the step, after step e) of sending a wireless signal by at least one wireless communication unit (30) to effect displaying of non-patient-specific data by the readable display.

A plurality of patient-assigned containers (10) may each assigned to a separate patient, and each patient-assigned containers (10) is responsive to a specific wireless signal, whereby the non-patient-specific data (22) is identical in each patient-assigned container (10).

Another embodiment of the invention is a system comprising:
- a patient-assigned container (10), which patient-assigned container (10) comprises:
   i. a housing (12) that defines a cavity (14) for receiving one or more prescriptions items, and
   ii. a wireless-activated readable display (20) attached to the housing (12), configured to:
      - receive and erasably store the patient-specific data, and
      - selectively display information,
         responsive to one or more wireless signals (22) specific to the patient-specific container (10);
- at least one wireless communication unit (30) configured to transmit signals to and/or receive signals from the wireless-activated readable display (20).

The system may further comprise a system interface (50), configured to co-ordinate communication between the patient-assigned container (10) and at least one wireless communication unit (30).

The system may be configured to perform a method as defined herein.

Another embodiment of the invention is a computer readable medium comprising a computer program comprising instructions for performing a method as defined herein.

### FIGURE LEGENDS

Embodiments of the present invention will now be described, by way of example only, with reference to the accompanying drawings, in which:
**FIG. 1****:** is a schematic illustration of a delivery system **100** comprising a patient-assigned container **10.**
**FIG. 2****:** is a schematic circuit diagram of a wireless-activated readable display **20.**
**FIG. 3****:** is a schematic circuit diagram of a wireless unit **30.**
**FIG. 4****:** depicts a flow chart indicating a procedure whereby a medical prescription for a patient is prepared and distributed to the patient *via* a patient-assigned container **10,** from the central pharmacy to the ward and finally to the patient.
**FIG. 5****:** depicts a flow chart indicating a procedure whereby a warning has been issued after the container **10** has left the pharmacy.
**FIG. 6****:** illustrates a method for preparing and distributing a medical prescription for a patient within a healthcare centre.
**FIG. 7****:** illustrates a method for preparing and distributing a medical prescription for a patient within a healthcare centre through a plurality of software modules.

### DETAILED DESCRIPTION OF INVENTION

Before the present system and method of the invention are described, it is to be understood that this invention is not limited to particular systems and methods or combinations described, since such systems and methods and combinations may, of course, vary. It is also to be understood that the terminology used herein is not intended to be limiting, since the scope of the present invention will be limited only by the appended claims.

As used herein, the singular forms "a", "an", and "the" include both singular and plural referents unless the context clearly dictates otherwise.

The terms "comprising", "comprises" and "comprised of" as used herein are synonymous with "including", "includes" or "containing", "contains", and are inclusive or open-ended and do not exclude additional, non-recited members, elements or method steps. It will be appreciated that the terms "comprising", "comprises" and "comprised of" as used herein comprise the terms "consisting of", "consists" and "consists of".

The recitation of numerical ranges by endpoints includes all numbers and fractions subsumed within the respective ranges, as well as the recited endpoints.

Whereas the terms "one or more" or "at least one", such as one or more or at least one member(s) of a group of members, is clear *per se,* by means of further exemplification, the term encompasses inter alia a reference to any one of said members, or to any two or more of said members, such as, e.g., any ≥3, ≥4, ≥5, ≥6 or ≥7 etc. of said members, and up to all said members.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

In the following passages, different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention, and form different embodiments, as would be understood by those in the art. For example, in the appended claims, any of the claimed embodiments can be used in any combination.

In the present description of the invention, reference is made to the accompanying drawings that form a part hereof, and in which are shown by way of illustration only of specific embodiments in which the invention may be practiced. Parenthesized or emboldened reference numerals affixed to respective elements merely exemplify the elements by way of example, with which it is not intended to limit the respective elements. It is to be understood that other embodiments may be utilised and structural or logical changes may be made without departing from the scope of the present invention. The following detailed description, therefore, is not to be taken in a limiting sense, and the scope of the present invention is defined by the appended claims.

The present invention provides a method and system for controlled distribution of prescriptions using a system of patient-assigned containers **10** each with a wirelessly-controlled programmable display and wireless communication units **30** for sending signals to program and individually change the display. The patient-assigned containers **10** essentially stay in communication with the wireless communication units **30,** to assist the administering nurse in the choosing the correct container at the patient bedside, and to warn of prescription changes.

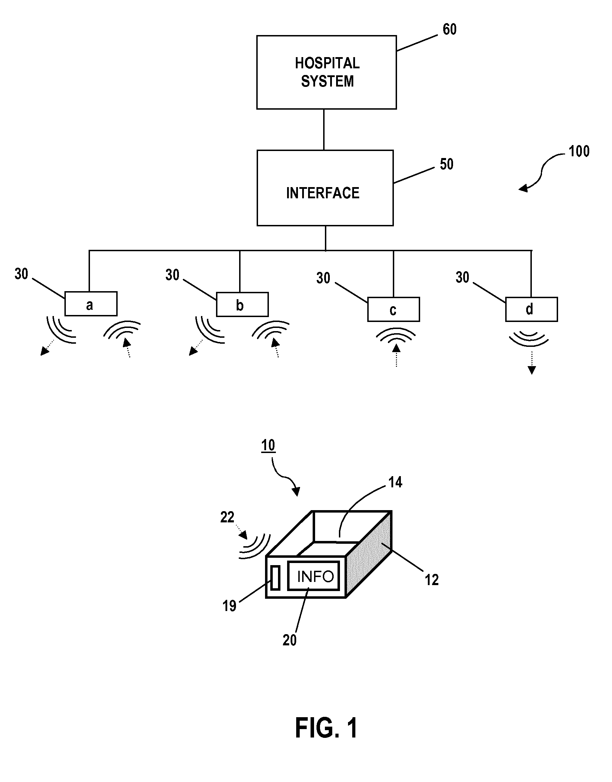

According to one embodiment of the invention and with reference to **FIG. 1****,** the delivery system **100** comprises a patient-assigned container **10** comprising:
a. a housing **12** that defines a cavity **14** for receiving one or more prescriptions items, and
b. a wireless-activated readable display **20** attached to the housing **12,** configured to:
   - receive and erasably store the patient-specific data, and
   - selectively display information,
      responsive to one or more wireless signals **22** specific to the patient-assigned container.

A patient in receipt of a prescription in a healthcare centre is assigned such a container **10.** The patient-assigned container **10** may be filled by the pharmacist, or an authorised assistant with medication or other medical supplies to be transported, such as from a central pharmacy, or other common distribution point, to a patient who may be located in different wards of a hospital, different units of a hospital, different floors of a hospital, different rooms of a hospital or the like. For example, the patient-assigned container **10** may be filled with the medication and other medical supplies that have been prescribed to the patient for consumption or use during a particular day, or other time frame (*e.g.* 2, 4, 6, 8, 10, 12, 14, 16, 18, 20 hours).

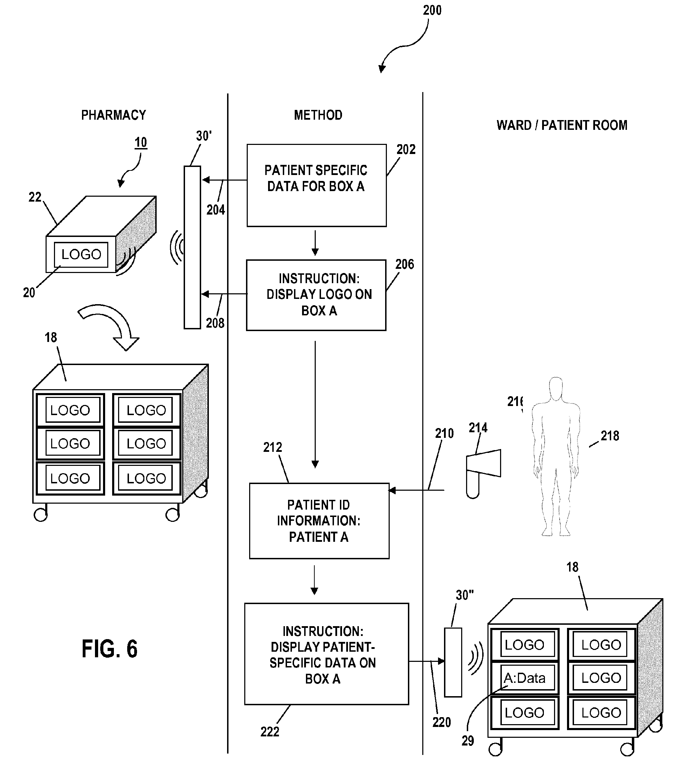

The patient-assigned container **10** includes a housing **12** configured to carry the medication or other medical supplies. In the illustrated embodiment, the housing has a generally rectangular shape that defines a cavity **14** therewithin for receiving the medication or other medical supplies. However, the housing **12** may be differently sized and shaped in other embodiments. The patient-assigned containers **10** are preferably uniform in size and multiple patient-assigned containers **10** can be loaded as drawers in a trolley **18** (shown in **FIG. 6****)** for transport around the healthcare centre.

In accordance with embodiments of the present invention, the container includes wireless-activated readable display **20** (also called "WAR display" **20** herein) carried by the container housing **12.** The wireless-activated readable display **20** may be permanently or dismountably attached to the container housing **12.** In the illustrated embodiment, the WAR display **20** is carried by and mounted to an external surface of a front face of the housing **12.** However, the WAR display **20** may be carried by different portions of the housing **12** in other embodiments if so desired. In order to facilitate reading of the display **20,** however, the WAR display **20** of one advantageous embodiment is carried by the patient-assigned container **10** such that the display is visible even after the medication and other medical supplies have been loaded in the cavity **14.** The WAR display **20** may be configured in various manners. For example, the WAR display **20** may be a liquid crystal display. Alternatively, the WAR display **20** may be comprised of electronic paper, thereby comprising an electronic ink display.

The WAR display **20** is disposed with a memory for storage of patient-specific data. Preferably the memory is erasable. Preferably, the WAR display **20** is configured to display one of several stored pages, which pages may be selectively displayed responsive to a wireless signal. The first page may contain, for instance, the name of the hospital, for example a logo. The first page provides anonymity to the patient while the patient-assigned container **10** is transported around the healthcare centre. The second page may contain, for instance, selected patient-specific data such as patient first name, surname, ward, room, bed etc. The third page may be held in reserve for other selected patient-specific data such as important messages (known also as "deltas") and/or extra functions.

Each WAR display **20** may contain a unique wireless code, allowing specific wireless communication with specific the wireless-activated readable display **20.** Accordingly, one WAR display **20** from a plurality of displays disposed on multiple containers may be specifically activated. A trolley **18** is typically used to transport multiple containers; activation of a single container from a loaded trolley **18** allows the health professional to select only the required display-activated container. Thus, a patient-specific container may be activated responsive to one or more wireless signals **22** specific to the patient-specific container.

The WAR display **20** is configured to receive instructions and/or data from a wireless communication unit **30** (see below). Besides a capability for receiving signals, the WAR display **20** may optionally contain a transmitter for transmitting a signal, for instance, to confirm transmission information (*e.g.* a checksum), to indicate a low battery, or to indicate an activity. Preferably, the WAR display **20** has both wireless transmission and receiving capability.

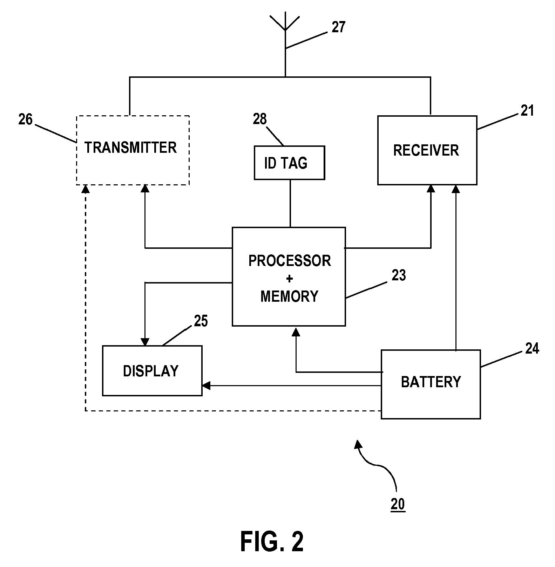

A configuration of an exemplary WAR display **20** is provided in **FIG. 2****.** It comprises a processor and memory **23** operatively connected to an electronic display **25** and receiver **21,** and optionally a transmitter **26** (indicated with dashed lines). The receiver **21** and transmitter **26** where present, are connected to an aerial **27.** A wireless unique ID tag, WUID tag, **28** is associated with the processor **23** that allows receipt and optionally transmission of signals specifically for a given WAR display **20.** The WUID tag determines the unique wireless code for a display **20** mentioned above. The components are powered by a power source such a battery **24** which may be one time use or rechargeable.

The patient-assigned container may further be provided with a container unique identification tag **19** (CUID tag) allowing identification of the container. The wireless code used to communicate specifically with the container may be linked with the CUID tag **19** in a database, thereby assigning a container to a particular wireless code. The CUID tag **19** may be any sort of ID tag, including but not limited to 1D barcode, 2D barcode, a radiofrequency ID tag and the like. The CUID tag **19** may be attached to the housing **12** or to the wireless-activated readable display **20.**

It will be appreciated that the system of the invention is designed for use with a plurality of patient-assigned containers **10,** each of which can be uniquely identified and communicated with by virtue of the CUID tag and WUID tag. Preferably, the non-patient-specific information is identical in each of the patient-assigned containers **10.**

Referring to **FIG. 1****,** the system further comprises at least one wireless communication unit **30** configured to transmit signals to the WAR display **20.** In particular, the wireless communication unit **30** is configured to transmit one or more wireless signals specific to the patient-specific container **10.** Where there is a plurality of containers, the wireless communication unit **30** is configured to transmit wireless signals specific to a patient-specific container **10,** thereby transferring different patient-specific data to each container, and separately instructing each container. Typically a wireless communication unit **30** is operatively connected to an existing medical computing system **60.**

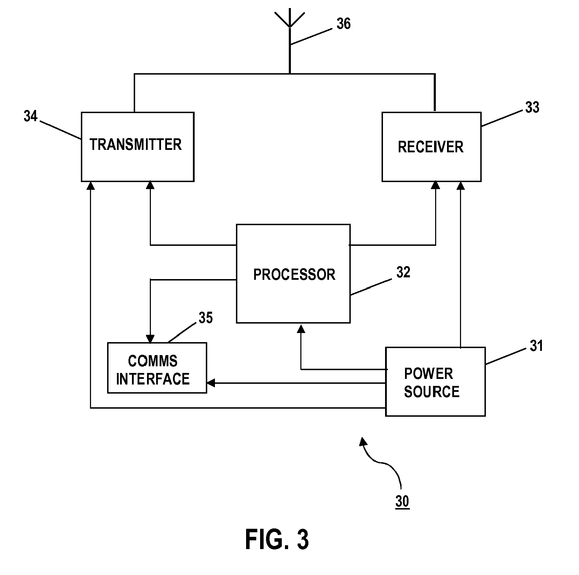

The wireless communication unit **30** may comprise a wireless transmitter (*e.g.* **30d),** or receiver (*e.g.* **30c)** or transceiver (*e.g.* **30a, b)** in the case of a combined transmitter and receiver. Preferably the wireless communication unit **30** is a transceiver. Wireless communication units **30** are known in the art, for example, commercial wireless routers using Wi-Fi or Bluetooth protocols, and the like. An exemplary circuit diagram is indicated in **FIG. 3****.** A typical wireless communication unit **30** contains an antenna **36** for transmitting and/or receiving a signal, a processor **32,** power supply **31,** a computer interface **35** (*e.g.* Ethernet or USB connection) and electronic circuits for transmission **34** and receipt **33** of data, operatively connected. Each wireless transmitter may be assigned an internet protocol (IP) address. Preferably, wireless communications are encrypted.

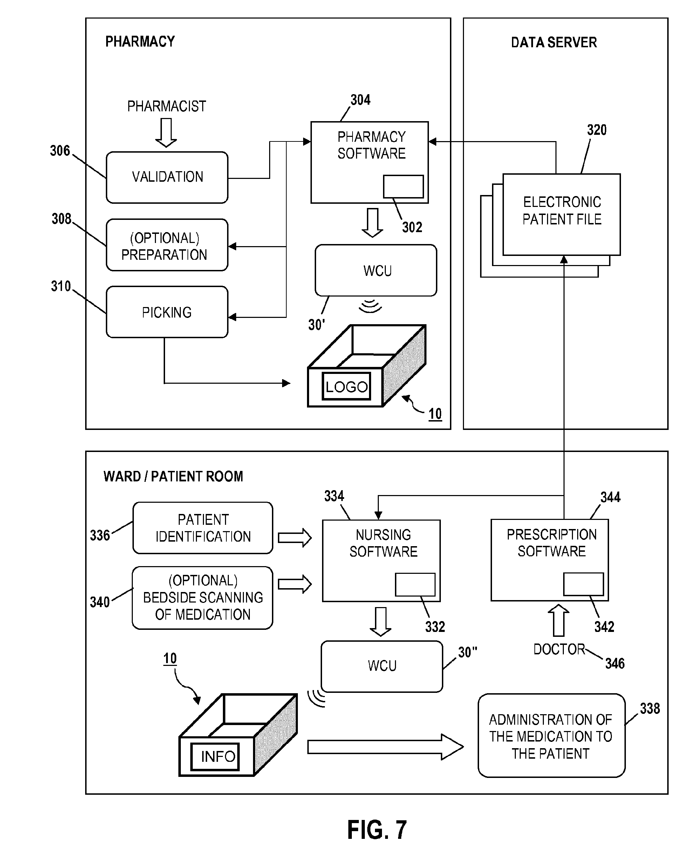

The system **100** may comprise at least one wireless communication unit **30,** preferably at least two wireless communication units **30,** and more preferably 3, 4, 5, 6, 7, 8, 9, 10 or more wireless communication units **30.** In particular, one or more wireless communication units **30** may be provided within the centralised pharmacy (central wireless communication unit **30',** shown in **FIG. 7****),** and one or more other wireless communication units **30** may be dispersed throughout the healthcare centre (peripheral wireless communication unit **30",** shown in **FIG. 7****).** Where there is a plurality of wireless communication units **30** distributed around a healthcare centre, the possibility arises to track the location of a patient-specific container **10.** A container **10** may be "pinged" for example by the wireless communication unit **30;** knowledge of the location of the wireless communication unit **30** that received a signal from the patient-specific container **10** allows the location of the patient-specific container **10** to be determined. Therefore, in case a container **10** has left the pharmacy, and a prescription has been updated, the location of the container **10** can be determined.

Referring to **FIG. 1****,** the system may further comprise a system interface **50** that comprises a computing device or a computer program, or both configured to co-ordinate communication between the patient-assigned container **10** and the at least one wireless communication units **30.** Preferably, the system interface **50** is configured to carry out a method of the invention which is described later below. It will be appreciated that the interface may comprise both a computing device and a computer program. The system interface **50** may interface with an existing medical computing system **60** installed by the healthcare centre.

Where the system interface **50** is a computing device **50** it may have a first communications interface to enable communication with the at least one wireless communication unit **30,** and a second communications interface to enable communication with an existing medical computing system **60** installed by the healthcare centre. Such first and second interfaces may be, for instance, an Ethernet connection, a WiFi connection, a Bluetooth connection, a universal serial bus (USB) connection and the like. A computing device **50** typically comprises a processor, a display, keyboard, hard-drive, communications interfaces, and memory as is well understood in the art.

Where the system interface **50** is a computer program, it may be provided as a plurality of modules that interface with the existing software modules running in a medical computing system **60** already installed by the health care centre. The wireless communication units **30** may connect to existing ports available in the existing medical computing system **60,** such as, for instance, an Ethernet connection, a WiFi connection, a Bluetooth connection, a universal serial bus (USB) connection and the like. Example of such modules are described herein below.

According to one aspect of the invention, a system comprises one or more of: a patient-assigned container **10,** at least one wireless communication unit **30,** and an interface **50.** Preferably, the system comprises a patient-assigned container **10,** and at least one wireless communication unit **30.** Even more preferably, the system comprises all three.

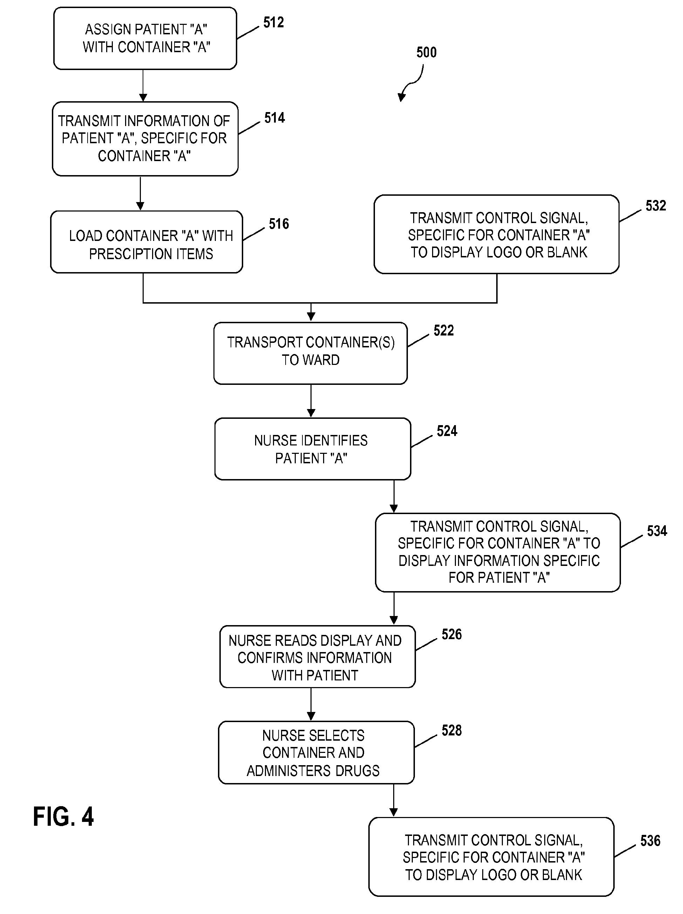

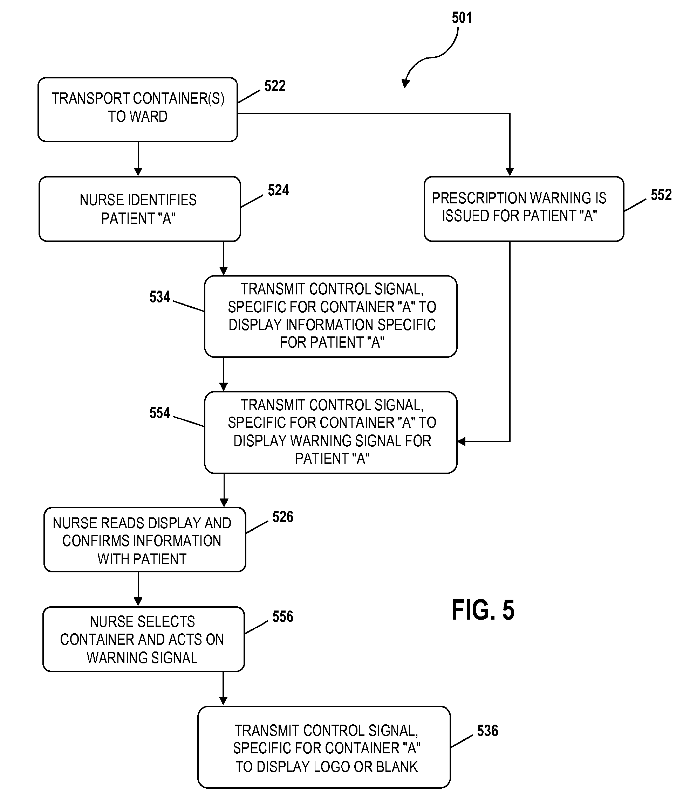

**FIG. 4** and **FIG. 5** provide non-limiting examples of methods for preparing and distributing a medical prescription for a patient within a healthcare centre according to the invention, preferably by using the system as described above.

In **FIG. 4****,** a possible procedure is shown in scheme **500,** whereby a medical prescription for a patient is prepared and distributed to the patient *via* a patient-assigned container **10,** from the central pharmacy to the ward and finally to the patient.

In a first set of steps, a container **10** is assigned to a patient **A** and loaded with prescription items prescribed to patient **A** by a doctor. This set of steps typically occurs in the central pharmacy of the healthcare centre. As mentioned earlier, such a container **10** comprises a cavity **14** for receiving one or more prescriptions items, and a wireless-activated readable display **20** attached to the container **10,** configured to receive and erasably store patient-specific information, and to selectively display information, responsive to one or more wireless signals specific to the patient-specific container **10.**

In a first step **512,** a container **10** is assigned to patient **A.** The patient-specific information of patient **A** is then transmitted in step **514,** for example through a wireless network to the container **10,** for example through the hospital system **60.** One or more wireless signals can be provided to the WAR display to effect receiving and storing of the patient-specific information. The patient-assigned container **10** is subsequently loaded in step **516** with the prescription items prescribed for patient **A,** for example by placing a patient-specific prescription (for a prescription period) into the cavity **14.**

Before the patient-assigned container **10** leaves the pharmacy, a control signal can be sent to the patient-assigned container **10** to display non-patient specific information on the display **20** in step **532,** thereby providing temporary anonymity to the patient-assigned container **10.** For example, one or more wireless signals can be provided to the WAR display **20** to effect displaying of non-patient specific information. Such non-patient specific information can be, for example, a blank screen, the hospital logo or an indication of the ward of patient **A.** The steps **512, 514, 516** and **532** can be performed for multiple patients **B to Z,** wherein each patient **B to Z** is assigned a container **B to Z** respectively, and wherein each container **B to Z** is loaded with the prescription items corresponding to patients **B to Z** respectively, and wherein each container **B to Z** is anonymously labeled with non-patient specific information on their displays **20** before transportation to the ward.

A second set of steps, *i.e.,* administering the prescription items to the corresponding patient **A** (as well as to patients **B to Z)** may be performed at a specific ward within the hospital. Once the patient-assigned container(s) **10** have been loaded, they can be transported to the ward in a trolley **18** in step **522,** optionally together with a plurality of other patient-specific containers assigned to other patients.

Once arrived in the ward, the nurse identifies the identity of patient **A** in step **524.** This can be performed through manually accessing the hospital computer system, or for example by scanning a bracelet **216** with a scanner **214** (as shown in **FIG. 6****).** Once patient **A** has been identified, the one or more wireless signals specific to the patient-specific container **10** assigned to patient **A** can transmit a control signal to effect display of the selective patient-specific information on the patient-specific container **10** in step **534.**

Optionally in step **526,** the nurse confirms whether the selective patient-specific information displayed corresponds with the identity of the patient. Subsequently, in step **528,** the nurse may select the patient-assigned container **10** displaying patient-specific information corresponding to patient **A,** for example to withdraw the prescription items from the patient-assigned container **10** and administer them to patient **A.**

In order to preserve anonymity of the other patients **B to Z,** the one or more wireless signals specific to the patient-assigned container **10** assigned to patient **A,** may transmit a control signal to effect display of the non-patient specific information (*e.g.* hospital logo) after the nurse has administered the prescription items to patient **A.**

**FIG. 5** shows an alternative scheme **501** for the case whereby a warning has been issued after the container **10** has left the pharmacy. Such a warning or "delta" may, for example, comprise a change in prescription items, a change in administration methods or a change in dosage.

Such a prescription warning can be issued for patient **A** in step **552** after the corresponding patient-assigned container has left the pharmacy in step **522** and before the nurse has identified patient **A** in step **524.** In that case, once the nurse has identified patient **A,** one or more wireless control signals will be transmitted to the patient-assigned container to display a warning signal (also referred to as a "delta") for patient **A** in step **554** optionally together with display information specific to patient **A** in step **534.** After confirming the information with the patient in step **526,** the nurse can select the patient-assigned container **10** in step **556** and act on the warning signal. For example, such a warning signal may tell the nurse that the prescription has changed. The nurse may need to consult the pharmacy and change the prescription items or instructions before administration.

After the nurse has acted upon the warning signal, the patient-assigned container **10** for patient **A** may be returned and a control signal may be transmitted to effect display of the non-patient specific information (*e.g.* hospital logo) in step **536.**

Referring to **FIG. 6****,** according to another embodiment of the invention, a method **200** for preparing and distributing a medical prescription for a patient within a healthcare centre comprises the steps:
a) obtaining patient-specific data **202;**
b) sending **204** patient-specific data **202** to at least one wireless communication unit **30** configured to transmit one or more wireless signals specific to a patient-assigned container **10,** which patient-assigned container **10** comprises:
   i. a housing **12** that defines a cavity **14** for receiving one or more prescriptions items, and
   ii. a wireless-activated readable display **20** attached to the housing **12,** configured to:
      - receive and erasably store the patient-specific data, and
      - selectively display information,
         responsive to one or more wireless signals **22** specific to the patient-specific container **10;**
c) sending **208** instruction **206** to at least one central wireless communication unit **30** to transmit a wireless signal to effect displaying of non-patient-specific data **22** by the readable display **20;**
d) receiving **210** patient identification data **212;**
e) sending **220** instruction **222** to at least one wireless communication unit **30** to effect displaying of patient-specific data **29** by the readable display **20** of the container that has been assigned to the patient identified by the identification data; thereby allowing a healthcare professional to confirm and identify the patient-assigned-container allotted to the patient.

The patient-assigned container **10,** at least one wireless communication unit **30,** and an interface **50** described in the system above, together with the features elaborated, may be used in the method.

The patient-specific data **202** comprises data that identifies a patient. As mentioned earlier, it includes one or more of the patient name (*e.g.* last name, first name, initials etc), hospital building, hospital ward, room number, bed number, date of birth. In **FIG. 6****,** the data is specific for container A and patient A. The patient-specific data **202** may also include an alert message ("delta"). Non-patient-specific data, to the contrary, is data from which a patient cannot be identified. Non-patient-specific data generally does not contain any alert information. Non-patient-specific data may be any, for instance, a blank screen, a black screen, a hospital logo, the time of loading, the day of loading, and the like. Patient specific data can typically be obtained from an electronic patient file **320** (shown in **FIG. 7****),** held on a central server which may be located at the healthcare centre.

In step b) method sends **204** patient specific data **202** to the patient-assigned container **10,** or more specifically to the WAR display **20** where it is stored in the memory. In **FIG. 6****,** the data is specific for container A and patient A. The patient specific data **202** typically contains the first name, surname, ward, room, and bed of the patient. The wireless signals contain a code specific to the patient-assigned container **10** that is recognised by the attached display **20.** The effect is that signals containing the code activate only one patient-assigned container **10,** while other displays of other containers that do not recognise the wireless code remain inactive. The container **10** is assigned to the patient, for instance, by a CUID tag **19** - a unique barcode or RFID tag, for instance - attached to the housing or to the readable display, which identifies the specific wireless code used to activate the display. The wireless code and patient specific data linked in a database. The WAR display **20** may transmit a confirmation that the data has been received. The wireless communication unit is preferably comprised in at least one central wireless communication unit **30'** having coverage in the centralized pharmacy. Typically, step b) is performed in the pharmacy, after which the container is loaded with prescription items.

In step c), an instruction **206** is sent **208** to the wireless communication unit **30** to transmit a wireless signal to the WAR display **20** to effect displaying of non-patient-specific data **22** by the WAR display **20.** Again, the wireless signal contains a code specific to the patient-assigned container **10** so that it activates only one patient-assigned container **10.** Where the readable display **20** has a three page memory, the non-patient-specific data **22** may be held in the first page, and the wireless signal instructs display only of the first page. The non-patient-specific data may be a hospital logo the time of loading, the day of loading, and the like. The wireless communication unit is preferably comprised in at least one central wireless communication unit **30'** having coverage in the centralized pharmacy. Typically, step c) is performed in the pharmacy, to anonymise each patient-assigned container **10** before it is loaded onto a trolley **18** and leaves the pharmacy.

In step d), the method receives patient identification data **212.** Typically this data will be obtained by the healthcare professional (*e.g.* nurse) whose duty it is to administer medication. The data is typically obtained by the nurse just prior to administration to ensure that the correct patient receives the right prescription. The identity of the patient may be ascertained, for instance, using a scanner **214,** as illustrated in **FIG. 6****,** configured to read a patient identification tag (PID tag) **216** disposed on or in the patient **218.** The PID tag **216** is unique to the patient. Various kinds of PID tags **216** exist in the art, for instance, an arm bracelet provided with a 1 D or 2D bar code or a radio frequency identification (RF-ID) tag, or an RF-ID tag provided as a subcutaneous implant. Scanners **214** are known in the art to read such PID tags employ a light source and light receiver in the case of a bar code tag, or a radio frequency source and radio receiver in the case of a RF-ID tag. It will be appreciated that patient may be identified by other means, such as by oral identification, by finger print, by face recognition and the like. It is also within the scope of the invention that identification proceeds *via* a manual input by the health care professional for instance, using a keyboard or touch screen. Typically, the identification proceeds *via* a software module of a existing medical computing system **60,** such as a nursing module.

In step e), an instruction **222** is sent **220** to a wireless communication units **30'** to transmit a wireless signal to the WAR display **20** to effect displaying of the stored patient-specific data **22** by the WAR display **20.** The wireless communication unit is preferably comprised in at least one peripheral wireless communication unit **30'** having coverage outside (and optionally inside) the centralized pharmacy. Patient-specific data may include first name, surname, ward, room, bed. Where the WAR display **20** has a three page memory, the patient-specific data **22** may be held in the second page, and the wireless signal instructs display only of the second page. Again, the wireless signal contains a code specific to the patient-assigned container **10** so that it activates only one patient-assigned container **10.** The data is read by the nurse, who is able to select the appropriate container, since it would be the only container displaying the patient-specific data.

The nurse would proceed to administer the prescription held by the patient-assigned container **10.** It is noted that, the system allows for the display of an alert message (see later below) in case a prescription has changed after the patient-assigned container **10** left the centralized pharmacy. This is described later below.

In an optional step f), a wireless signal is sent by the wireless communication units **30'** to effect displaying of non-patient-specific data by the WAR display. Again, the wireless signal contains a code specific to the patient-assigned container **10** so that it activates a specific patient-assigned container **10.** Where the WAR display **20** has a three page memory, the non-patient-specific data **22** may be held in the first page, and the wireless signal instructs display only of the first page. The non-patient-specific data may be a hospital logo the time of loading, the day of loading, and the like. By reverting the WAR display back to non-patient-specific data, the anonymity of each patient-assigned container is assured. Step f) may be performed after the nurse has administered the prescription, or after a preset period of time.

As mentioned earlier, the system allows for the display of an alert message in case a prescription has changed after the patient-assigned container **10** left the centralised pharmacy. Therefore, in step c), the patient-specific data may be a stored alert message (also known as a "delta"). Where the WAR display **20** has a three page memory, the patient-specific data **22** containing the alert may be stored in page three, and the wireless signal instructs display only of the third page. Again, the wireless signal contains a code specific to the patient-assigned container **10** so that it activates only one patient-assigned container **10.** The data is read by the nurse, who is able to select the appropriate container, since it would be the only container displaying the patient-specific data; she would check the prescription with the pharmacy. If necessary, the prescription might be returned to the pharmacy.

Optionally, the nurse may identify the medication, before or after or before and after administration, using for instance, 1 D or 2D barcode scanning.

One aspect of the invention relates to a computer readable medium comprising a computer program comprising instructions for performing a method as described herein. The computer readable medium may be an optical storage medium, a solid-state memory (*e.g.* flash memory), a hard drive, a cloud storage device, a cloud computing device and the like.

The present invention provides plurality of advantages. In case of a manual or a semi automatic container loading process in the central pharmacy, the wireless-activated readable display can be instructed to display patient-specific information such as name, surname, room, and bed etc. This gives an extra visual control during this loading process.

Once the container has been loaded, it is transported to the ward on a trolley. The wireless-activated readable display is instructed to display non-patient-specific information. No patient name is visible; this guarantees the necessary confidentiality of the medical information about the patient. No unauthorised persons would be able to see medication prescribed to the patient must take, which would otherwise compromise confidentiality. The containers are stored on a medication trolley on the ward; since all the displays indicate non-patient-specific information, no names are visible.

Prior to the administration of the medicines, the nurse identifies the patient, then his patient file will open via the hospital compute system **60.** When opening the patient file, the wireless communication unit **30** will send a signal to the WAR display **20** of the container to effect the display of patient-specific information (*e.g.* name). The nurse will recognize directly the container for that patient in the trolley, since it will be the only container with a different display. This functionality will reduce mix-ups of medicine from different patients and will increase patient safety during the medicine administration.

An important problem in the central distribution of medicines is managing the "deltas". A "delta" will occur when a doctor changes the prescription after the container **10** has been loaded in the pharmacy and before the administration of the medicine has taken place. The system of the invention can detect through the interface which wireless communication unit **30** is communicating with the WAR display **20** on the container **10.** Through the system, the location of the container **10** can be determined whether it is in pharmacy, in transit or on the ward. If the delta occurs when the medication container is still in the pharmacy, then the medication can be adjusted conform the new prescription. If the "delta" occurs when the medication container has already left the Pharmacy, when it is in transit or already on the ward, then an extra "delta" symbol may be displayed on the container **10.** The "delta" symbol may be a triangle, also the symbol for danger, and this will warn the nurse that she must check thoroughly the new prescription before the administration of the medicines. This process will increase patient safety during the medicine administration by warning for "deltas" in a centralized medication distribution system.

Healthcare centres generally have their own medical computing system **60** that controls the distribution of patient information to doctors, nurses, pharmacies and administrators. The present method of the invention may be implemented as a plurality of software modules that interface with an existing medical computing system **60** of a healthcare centre. An exemplary configuration of modules according to the invention is provided in **FIG. 7****.**

A pharmacy module **302** of the invention interfaces with the existing pharmacy software **304.** The existing pharmacy software **304** may be used in the healthcare centre to access an electronic patient file **320,** and in the centralized pharmacy to validate prescriptions **306,** and optionally prepare **308,** pick **310** and pack prescription items, often robotically. A pharmacy module **302** of the invention receives patient-specific data (*e.g.* name, location), and also assigns a unique container **10** to that patient. The pharmacy module **302** also sends patient-specific data wirelessly using a centrally-located wireless communication unit (WCU) **30'** to that unique container **10** in the pharmacy.

A nursing module **332** of the invention interfaces with the existing nursing software. The existing nursing software **334** may be used in the healthcare centre to access an electronic patient file **320,** and in the ward or patient room to display patient information as the patient bed side. A nursing module **332** of the invention receives patient identification data (*e.g.* by manual input, by scanning, face recognition etc). Following identification **336,** it also sends instructions to the peripherally-located wireless communication unit (WCU) **30"** to effect the display of patient-specific data by the readable display **20** of the container **10** that has been assigned to the patient identified by the identification data. Providing there are no alert messages, the container showing the patient-specific data is selected and the medication is administered **338** by the nurse. Optionally, the nurse may identify **340** the medication, before or after or before and after administration, using for instance, barcode scanning.

A prescription module **342** of the invention interfaces with the existing prescription software **344.** The existing prescription software **344** may be used in the healthcare centre to access an electronic patient file **320,** and in the ward or patient room to allow a doctor **336** to enter prescription information. A prescription module **342** of the invention checks for updates to the prescription that may arise after the patient-assigned container has left the pharmacy. It then alerts the nursing module **332** to send instructions to the peripherally-located wireless communication unit (WCU) **30"** to effect the display of patient-specific data, after patient identification, by the readable display **20** of the container **10** that has been assigned to the patient identified by the identification data, which patient-specific data is an alert.

## Claims

1. A method (200) for computer assisted distribution of a medical prescription for a patient within a healthcare centre comprising the steps:
a) obtaining patient-specific data (202);
b) sending (204) patient-specific data (202) to at least one wireless communication unit (30) configured to transmit one or more wireless signals specific to a patient-assigned container (10), which patient-assigned container (10) comprises:
i. a housing (12) that defines a cavity (14) for receiving one or more prescriptions items, and
ii. a wireless-activated readable display (20) attached to the housing (12), configured to:
- receive and erasably store the patient-specific data, and
- selectively display information,
responsive to one or more wireless signals (22) specific to the patient-specific container (10);
c) sending (208) instruction (206) to at least one wireless communication unit (30) to transmit a wireless signal to effect displaying of non-patient-specific data (22) by the readable display (20);
d) receiving (210) patient identification data (212);
e) sending (220) instruction (222) to at least one wireless communication unit (30) to effect displaying of patient-specific data (24) by the readable display (20) of the container (10) that has been assigned to the patient identified by the identification data (212);
thereby allowing a healthcare professional to confirm and identify the patient-assigned-container allotted to the patient prior to administration of the prescription items.

2. Method according to claim 1, wherein the patient-assigned container (10) is identified using a container unique identification tag, CUID tag (19), attached to the container (10), and which is assigned to the patient.

3. Method according to claim 2, wherein the CUID tag (19), is a 1 D or 2D barcode, or a radiofrequency identification tag.

4. Method according to any of claims 1 to 3, wherein the patient specific data displayed in step e) comprises the patient name, and one or more of hospital building, hospital ward, room number, bed number, and date of birth.

5. Method according to any of claims 1 to 3, further comprising a step after step c) of receiving an indication that the prescription of the patient has changed, and wherein the patient-specific data (24) displayed in step e) is a stored alert message.

6. Method according to any of claims 1 to 5, wherein the patient identification data (212) is obtained in step d) using:
- a scanner (214) configured to read a patient identification tag, PID tag, (216) disposed on or in the patient (218),
- face recognition,
- finger print recognition, or
- manual input by a healthcare professional.

7. Method according to any of claims 1 to 6, further comprising the step, after step e) of sending a wireless signal by at least one wireless communication unit (30) to effect displaying of non-patient-specific data by the readable display.

8. Method according to any of claims 1 to 7, wherein a plurality of patient-assigned containers (10) is each assigned to a separate patient, and each patient-assigned containers (10) is responsive to a specific wireless signal, whereby the non-patient-specific data (22) is identical in each patient-assigned container (10).

9. A system comprising:
- a patient-assigned container (10), which patient-assigned container (10) comprises:
i. a housing (12) that defines a cavity (14) for receiving one or more prescriptions items, and
ii. a wireless-activated readable display (20) attached to the housing (12), configured to:
- receive and erasably store the patient-specific data, and
- selectively display information,
responsive to one or more wireless signals (22) specific to the patient-specific container (10);
- at least one wireless communication unit (30) configured to transmit signals to and/or receive signals from the wireless-activated readable display (20).

10. A system according to claim 9, further comprising a system interface (50), configured to co-ordinate communication between the patient-assigned container (10) and at least one wireless communication unit (30).

11. System according to claim 9 or 10, configured to perform a method as defined in any of claims 1 to 8.

12. Computer readable medium comprising a computer program comprising instructions for performing a method according to any of claims 1 to 8.
